# EUROPEAN PATENT APPLICATION

(11) **EP 1 795 207 A1**
(43) Date of publication of application: **13.06.2007**
(21) Application number: 05741464.1
(22) Date of filing: 17.05.2005
(51) Int. Cl.: A61K 47/36, A61K 9/24, A61K 9/28, A61K 31/137, A61K 45/00, A61K 47/02, A61K 47/38

(54) **SOLID PHARMACEUTICAL PREPARATION**

(30) Priority: 01.10.2004 JP 2004290369
(71) Applicant: NIPPON ZOKI PHARMACEUTICAL CO. LTD., Osaka-shi, Osaka 541-0046 (JP)
(72) Inventor: ISHITSUBO, N., NIPPON ZOKI PHARMACEUTICAL CO., LTD, Ono-shi, Hyogo 675-1363 (JP)
(74) Representative: HOFFMANN EITLE
(86) International application number: PCT/JP2005/008935
(87) International publication number: WO 2006/038339

(57) **Abstract**

To provide a sustained-release solid pharmaceutical preparation having a quick-release part and a sustained-release part and stably having an excellent quick releasing characteristic with little pH dependency in an initial dissolution.

The present invention relates to that, in a preparation containing a medical ingredient as an effective ingredient, particularly active analgesic ingredient, a sustained-release solid pharmaceutical preparation which is **characterized in that** it is a solid pharmaceutical form having a quick-release part and a sustained-release part and contains effective ingredient in both parts and the quick-release part contains a partly pregelatinized starch and a low substituted hydroxypropylcellulose as additives. In the preparation of the present invention, stable and quick initial dissolution behavior being independent upon pH is achieved even when some sustained-release part is contaminated in the quick-release part due to the difference in the tabletting method for multi-layered tablets. Furthermore, the preparation is practical as a preparation having a sufficient hardness in view of necessity that abrasion, breakage, crack, etc. are not generated when the tablet is coated.

## Description

### Technical Field

The present invention relates to a solid pharmaceutical preparation having a quick-release part and a sustained-release part.

### Background Art

As a result of progresses in the technique of pharmaceutical preparations at present, technical developments for making the sustained-release preparations of various drugs has been done for a purpose of improvement in compliance of patients. As compared with conventional quick-release preparations, sustained-release preparations can sustain the pharmaceutical effect, and so they have many advantages in efficacy and safety. For example, the sustained-release preparations may effectively bring out a latent effect of a drug, and they can decrease the number of administrations and reduce side effect or toxicity.

However, in analgesics, an immediate action is also demanded. Therefore, such immediate action expressing the analgesic effect by achieving an effective blood level quickly after administration is requested. As a preparation having both characteristics of immediate and sustained actions, a two-layered tablet having quick-release part and sustained-release part or a sustained-release preparation where slowly acting granules are mixed with a quickly soluble part has been developed.

In a sustained-release preparation, it is regarded as an excellent preparation where the preparation is hardly influenced by food or physiological factors of a digestive tract so that drug level in blood may be sustained in an-appropriate level for proper time, and also where variation in and among private person(s) is as little as possible. To achieve a stable blood level in oral administrations, it is necessary to formulate a pharmaceutical preparation that is hardly affected by physiological characteristics, particularly pH, in a digestive tract. For example, in the case of multi-layered tablets comprising a quick-release part and a sustained-release part, when the manufacturing methods of tablets are different, it may be occurred that the initial dissolution is retarded or the release of a medical ingredient greatly varies by pH of a dissolution medium. Such a phenomenon may be generated when tablets are manufactured by a continuous tabletting machine for a large-scale production and that is presumably due to contamination of the sustained-release component into the quick-release part caused by adhesion and remaining of the sustained-release part to and in the inner area of the machine.

With regard to an analgesic ingredient as an effective ingredient of the solid pharmaceutical preparation of the present invention, it has been also investigated for a sustained-release preparation where an effective blood level is achieved immediately after administration and also the pharmaceutical effect may be sustained for long time. For example, there is a disclosure for a multi-layered preparation containing at least one kind of opioid analgesics and having a form comprising a quickly releasing part and a slowly releasing part (Japanese Patent Laid-Open No. 10/251,149). However, in this document there is no description at all regarding a sustained-release preparation having an excellent releasing characteristic with little pH-dependency in the initial opioid dissolution and also a technique for achieving a pharmaceutical preparation as such.

### Disclosure of the Invention

An object of the present invention is to provide a sustained solid pharmaceutical preparation containing a drug, particularly, an active analgesic ingredient as an effective ingredient, and stably having an excellent quick releasing characteristic with little pH-dependency in the initial dissolution and also having a sufficient hardness for making into pharmaceutical preparation.

In order to select a preparation showing an excellent releasing characteristic with little pH dependency, preparations were formulated by combing various additives such as binder, disintegrating agent, filler and lubricant. Thus, by taking variation and difference in pH in digestive tract into consideration, a releasing characteristic when water or a solution of pH 1.2, pH 4.0 or pH 6.8 was used as a dissolution medium was repeatedly examined. Further, in the selection of such additives, studies for the manufacture of pharmaceutical preparations were conducted so that an essential condition where not only an excellent releasing characteristic is achieved but also strength (hardness) demanded as a solid preparation can be maintained were satisfied. Further, investigation was conducted for developing a preparation where a stable and quick dissolution characteristic is achieved even when actual production is carried out continuously and in large quantities using a continuous tabletting machine for layered tablets (in other words, even when some slowly releasing component is contaminated in a quickly releasing part).

The present inventor has conducted an intensive study for making an oral preparation sustained-release. As a result, he has found that, by preparing a pharmaceutical formulation where a medical ingredient is contained in each of quick-release part and sustained-release part and a partly pregelatinized starch and a low substituted hydroxypropylcellulose are used particularly in the quick-release part in order to achieve the pharmaceutical effect immediately after administration and to maintain the pharmaceutical effect as such, it is now possible to manufacture a pharmaceutical preparation having a stable quick releasing characteristic with little pH dependency in the initial dissolution without being effected by a tabletting method and the like. Thus, the present invention has been accomplished.

The solid pharmaceutical preparation according to the present invention is a sustained release preparation where an effective blood level is achieved immediately after administration and the pharmaceutical effect may be maintained for long time thereafter. Since it has a quick releasing characteristic with little pH dependency in its initial dissolution, it is very highly useful as a sustained release preparation giving a stable drug level in blood without being affected by variation and difference in pH in a digestive tract. In addition, the preparation of the present invention shows a stable and pH-independent quick initial dissolution behavior even if a situation of some contamination of a sustained-release part in a quick-release part is resulted due to the difference in a tabletting method for multi-layered tablets and, further, in view of the necessity that abrasion, breakage, crack, etc. are not generated in coating the tablets, the preparation is practical enough having a sufficient hardness.

### Brief Description of the Drawings

Fig. 1 is a graph which shows the result of dissolution test (until 120 seconds from the initiation of dissolution) of the solid pharmaceutical preparation of the present invention shown in Example (containing 100 mg of tramadol hydrochloride per tablet).
Fig. 2 is a graph which shows the result of dissolution test (until 120 seconds from the initiation of dissolution) of the solid pharmaceutical preparation for comparison shown in Comparative Example (containing 100 mg of tramadol hydrochloride per tablet).
Fig. 3 is a graph which shows the result of dissolution test (until 12 hours from the initiation of dissolution) of the solid pharmaceutical preparation of the present invention shown in Example (containing 100 mg of tramadol hydrochloride per tablet).

### Best Mode for Carrying Out the Invention

The present invention relates to that, in a preparation containing a medical ingredient as an effective ingredient, particularly active analgesic ingredient, a sustained-release solid pharmaceutical preparation which is characterized in that it is a solid pharmaceutical form having a quick-release part and a sustained-release part and contains effective ingredient in both parts and the quick-release part contains a partly pregelatinized starch and a low substituted hydroxypropylcellulose as additives. A preferred solid pharmaceutical preparation according to the present invention is characterized in that the quick-release part and the sustained-release part contain tramadol or a pharmaceutically acceptable salt thereof as an effective ingredient and a dissolution rate of the effective ingredient from the solid pharmaceutical preparation in accordance with a dissolution test by the method 2 (Paddle method) of Dissolution Test of General Tests, Processes and Apparatus in the Japanese Pharmacopoeia where a dissolution test is conducted at fluid temperature of 37°C, with dissolution medium of 900 mL and at 50 rpm is 30 to 50% by weight after 15 minutes, 40 to 60% by weight after 1 hour, 50 to 70% by weight after 2 hours, 60 to 80% by weight after 4 hours and 70 to 90% by weight at 6 hours.

Various kinds of medical ingredients are able to be applied to the solid pharmaceutical preparation of the present invention and an active analgesic ingredient is particularly preferred. There is no particular limitation for the active analgesic ingredient where its examples are tramadol, pentazocine and buprenorphine and any of them may be used in a pharmaceutically acceptable salt thereof. Particularly preferred active analgesic ingredient is tramadol. Tramadol is a non-narcotic analgesic which is positioned between a strong narcotic analgesic with an indication of cancerous pain, etc. and a non-steroidal anti-inflammatory drugs (NSAID) with an indication of light pain such as headache and arthralgia. As compared with a strong opioid such as morphine, it has a medical usefulness as a drug having a low frequency of side effect to respiratory, circulatory and digestive systems and hardly causing resistance, physical dependence, abuse, etc. In tramadol, a pharmaceutically acceptable acid addition salt is also able to be used without particular limitation and its examples are inorganic acid salt such as hydrochloride, sulfate, nitrate, phosphate, hydrofluoride and hydrobromide and organic acid salt such as acetate, tartrate, lactate, citrate, fumarate, maleate, succinate, methanesulfonate, benzenesulfonate, toluenesulfonate, naphthalenesulfonate and camphorsulfonate. Particularly preferred one is a salt of tramadol with hydrochloric acid (tramadol hydrochloride) which has been available in the market and widely used in clinic as an analgesic agent. Stereoisomers, hydrate and solvate of tramadol are also included in tramadol which is able to be used as an effective ingredient in the solid pharmaceutical preparation of the present invention.

The compounding amount of the medical ingredient in the solid pharmaceutical preparation of the present invention is not particularly limited but may be appropriately selected depending upon the size of the tablet, etc. In the case of an active analgesic ingredient such as tramadol hydrochloride, it is usually appropriate to be 15 to 70% by weight, preferably 15 to 65% by weight and, more preferably, 20 to 55% by weight to 100% by weight of the quick-release part of the solid pharmaceutical preparation. When the compounding amount is too small, there may be a case where the size of the tablet is to be made big for achieving a sufficient pharmaceutical effect. Meanwhile, when it is too much, compounding of other additive is restricted whereby inconvenience in drug design may be resulted. Incidentally, the solid pharmaceutical preparation of the present invention has a quick-release part and a sustained-release part and it is fundamental to comprise those two layers although it is also possible that other layer is appropriately added. Although the mass ratio of the medical ingredient contained in each of the quick-release part and the sustained-release part is not particularly limited, it is appropriate when the ratio of the quick-release part to the sustained-release part is from 1:1 to 1:5 in the case of an active analgesic ingredient such as tramadol hydrochloride.

The characteristic of the solid pharmaceutical preparation of the present invention is that, in the initial dissolution of the medical ingredient, a quick releasing characteristic with little pH dependency is available. Since that is also a characteristic of the composition of the quick-release part, additives used in the quick-release part will be illustrated in detail below.

The partly pregelatinized starch used as an additive for the quick-release part of the solid pharmaceutical preparation of the present invention is a product where corn starch is heated with water under ordinary pressure or increased pressure and the resulting starch granules which are partly pregelatinized are dried. That which is listed in "Japanese Pharmaceutical Excipients 2003" (edited by Japan Pharmaceutical Excipients Council; published by Yakuji Nippo, Ltd.) may be used and that is commercially available. In the present invention, it is appropriate to be about 20 to 70% by weight and, preferably, 25 to 55% by weight of the partly pregelatinized starch to 100% by weight of the quick-release part of the solid pharmaceutical preparation.

The low substituted hydroxypropylcellulose which is used as another additive for the quick-release part of the solid pharmaceutical preparation of the present invention is a low substituted hydroxypropyl-ether of cellulose. That is commercially available and that which is listed in the Japanese Pharmacopoeia (15th Edition) is able to be used. In the present invention, amount of the low substituted hydroxypropylcellulose to 100% by weight of the quick-release part of the solid pharmaceutical preparation is preferably about 5 to 25% by weight and, more preferably, 5 to 20% by weight.

When a synthetic aluminum silicate is compounded with the quick-release part of the solid pharmaceutical preparation of the present invention in addition to the above components, it is more advantageous in improving the physical quality. Thus, tablets are preferred as the dosage form of the solid pharmaceutical preparation of the present invention and an appropriate strength as tablets is needed. However, only the partly pregelatinized starch and the low substituted hydroxypropylcellulose which are additives for achieving the advantage of the present invention may be sometimes insufficient in terms of hardness or may result in a capping (a phenomenon where tablets are cracked into a lens-like shape). Therefore, when synthetic aluminum silicate is added, it is now possible to prepare a pharmaceutical preparation having a necessary hardness. Moreover, as a result of improvement in hardness, it is also possible to improve abrasion and breakage as well as crack which are generated in coating the tablets whereby it is now also possible to make the solid pharmaceutical preparation of the present invention into coated tablets. Although there is no particular limitation for the compounding amount of synthetic aluminum silicate, it is possible to achieve a desired hardness by addition of usually about 1 to 15% by weight and, preferably, 5 to 10% by weight to 100% by weight of the quick-release part of the solid pharmaceutical preparation.

Besides the above, the quick-release part of the solid pharmaceutical preparation of the present invention may contain various additives used for the manufacture of common pharmaceutical preparations so far as that does not deteriorate the advantages of the invention. Examples of the additives as such are disintegrating agent, binder, corrigent, foaming agent, perfume, lubricant and coloring agent and they may be appropriately added depending upon the object.

The sustained-release part of the solid pharmaceutical preparation of the present invention may be prepared using the conventional sustained-release base and, for example, a gel-former which is able to control the release of a medical ingredient by forming hydrogel upon contacting to water may be utilized. Examples of the preferred gel-former are cellulose derivative such as hydroxypropylcellulose, methylcellulose, hydroxypropylmethylcellulose and sodium carboxymethylcellulose and carboxyvinyl-polymer. It is also possible to prepare a sustained-release part of the solid pharmaceutical preparation of the present invention by an appropriate addition of a lubricant such as magnesium stearate or the like. Further, the same as in the above-mentioned quick-release part, various additives used for the manufacture of common preparations such as disintegrating agent, binder, corrigent, foaming agent, lubricant and coloring agent may also be appropriately added to the sustained-release part depending upon the object.

The solid pharmaceutical preparation of the present invention having the above-mentioned quick-release part and sustained-release part may be coated if necessary. Depending upon the type of the medical ingredient, there is a case where coating is preferred with a purpose of masking of bitter taste or irritation, stabilization of the main ingredient, etc. When a coating is done, damage and abrasion of tablets hardly take place and that is convenient for transportation and packaging. As mentioned above, the quick-release part of the solid pharmaceutical preparation of the present invention *per se* has a preferred releasing characteristic. Therefore, when a special coating for a purpose of sustained-release is used, the preferred releasing characteristic may , be- disturbed. Accordingly, it is preferred in the present invention to conduct a conventional quickly dissolving film coating.

### Examples

The present invention will now be specifically illustrated by way of the following Examples although the present invention is not limited thereto at all.

Table 1 shows each amount of components per tablet for the preparations of Examples prepared by the formulation of the solid pharmaceutical preparation of the present invention and of Comparative Example prepared by the formulation where additives are different from the solid pharmaceutical preparation of the present invention (refer to Japanese Patent Application No. 2004/288,138). According to the following preparing method, two-layered tablets of tramadol hydrochloride of Examples (containing 100, 75 and 50 mg of tramadol hydrochloride per tablet) and of Comparative Example (containing 100 mg of tramadol hydrochloride per tablet) comprising the compositions shown in Table 1 were prepared.

**Table 1**

| Formulation (mg/tablet) | Examples | | | Comparative Example |
|---|---|---|---|---|
| | 100-mg Tablet | 75-mg Tablet | 50-mg Tablet | |
| Quick-Release Part | | | | |
| Tramadol hydrochloride | 35.0 | 26.25 | 17.5 | 35.0 |
| Erythritol | - | - | - | 12.3 |
| Microcrystalline cellulose | - | - | - | 4.0 |
| Synthetic aluminum silicate | 6.0 | 6.0 | 6.0 | 4.0 |
| Crospovidone | - | - | - | 14.0 |
| Partly pregelatinized starch | 21.3 | 26.55 | 35.3 | - |
| Low substituted hydroxypropylcellulose | 7.0 | 10.5 | 10.5 | - |
| Magnesium stearate | 0.7 | 0.7 | 0.7 | 0.7 |
| Subtotal | 70.0 | 70.0 | 70.0 | 70.0 |

| Sustained-Release Part | | | | |
|---|---|---|---|---|
| Tramadol hydrochloride | 65.0 | 48.75 | 32.5 | 65.0 |
| Lactose | - | 18.25 | 36.5 | - |
| Hydroxypropylcellulose | 120.0 | 120.0 | 120.0 | 133.0 |
| Carboxyvinyl-polymer | 27.0 | 27.0 | 27.0 | 25.0 |
| Carmellose sodium | 6.0 | 4.0 | 2.0 | 5.0 |
| Magnesium stearate | 2.0 | 2.0 | 2.0 | 2.0 |
| Subtotal | 220.0 | 220.0 | 220.0 | 230.0 |
| Grand Total | 290.0 | 290.0 | 290.0 | 300.0 |

### [Examples]

Tramadol hydrochloride (350 g), 143 g of partly pregelatinized starch and 60 g of synthetic aluminum silicate were mixed, disintegrated and granulated using pure water. Low substituted hydroxypropylcellulose (70 g) and 70 g of partly pregelatinized starch were added to and mixed with the above-prepared granules and then 7 g of magnesium stearate was added thereto and mixed therewith to give quick-release granules. On the other hand, 650 g of tramadol hydrochloride, 1,200 g of hydroxypropylcellulose and 60 g of carmellose sodium were mixed, disintegrated and granulated with pure water. Carboxyvinyl-polymer (270 g) was added to and mixed with the above granules and then 20 g of magnesium stearate was added thereto and mixed therewith to give sustained-release granules. The resulting quick-release granules and sustained-release granules were made into tablets using a continuous tabletting machine for layered tablets (HT-AP38-LII; manufactured by Hata Iron Works Co., Ltd.) to prepare double-layered tablets each containing 100 mg of tramadol hydrochloride. With regard to the preparations where tramadol hydrochloride per tablet was 75 mg and 50 mg, they were manufactured by the same manner as above except that, in the manufacture of the above sustained-release part, lactose was further added to tramadol hydrochloride, hydroxypropylcellulose and carmellose sodium followed by mixing.

### [Comparative Example]

Tramadol hydrochloride (350 g), 123 g of erythritol, 40 g of microcrystalline cellulose and 40 g of synthetic aluminum silicate were mixed, disintegrated and granulated with pure water. Crospovidone (140 g) was added to and mixed with the above granules and then 7 g of magnesium stearate was added thereto and mixed therewith to give quick-release granules. With regard to sustained-release granules, they were manufactured by the same manner as in the above Examples according to the compounding amount shown in Table 1. The quick-release granules and the sustained-released granules prepared as above were made into tablets using a continuous tabletting machine to give two-layered tablets of tramadol hydrochloride.

### [Test Example: Dissolution Test]

The two-layered tablets of tramadol hydrochloride (100 mg tramadol hydrochloride/tablet) manufactured in the above Example and Comparative Example were subjected to a dissolution test in accordance with the method 2 (Paddle method) of Dissolution Test of General Tests, Processes and Apparatus in the Japanese Pharmacopoeia (hereinafter, abbreviated as JP). With regard to the dissolution medium, the first fluid (pH 1.2) of Disintegration Test, General Tests, Processes and Apparatus of the JP, water, an acetic acid/sodium acetate buffer (0.05 mol/L, pH 4.0) and a two-fold diluted solution of a phosphate buffer (pH 6.8) in Reagents, Test Solutions of the JP were used.

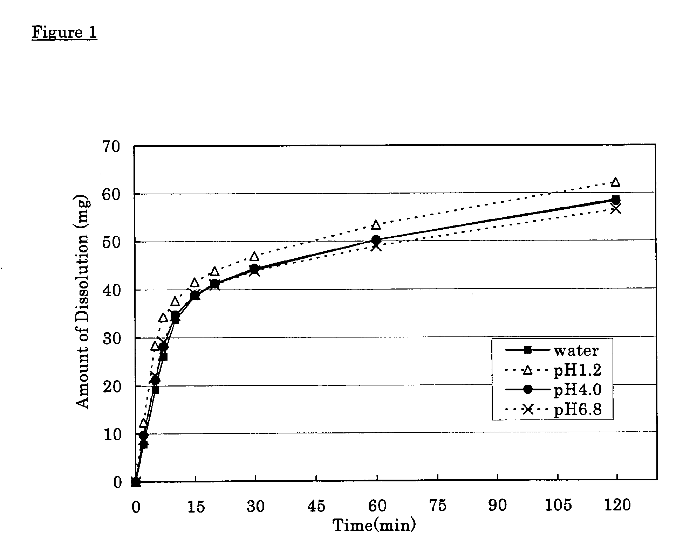

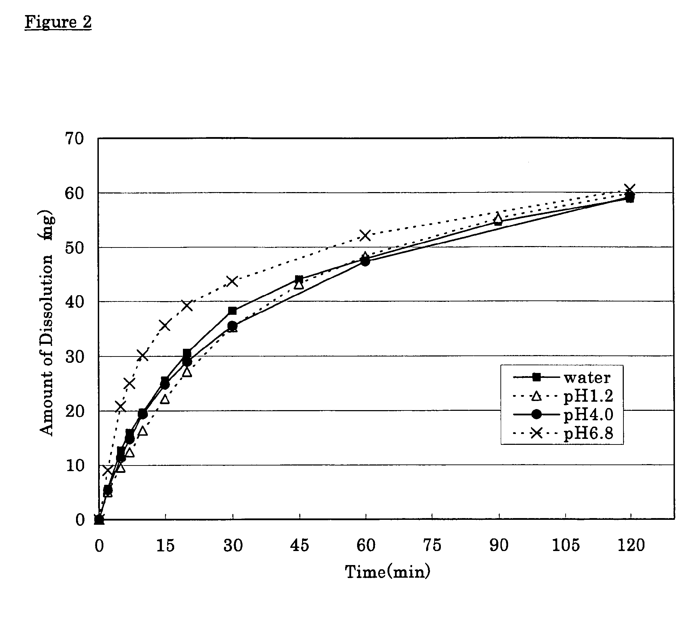

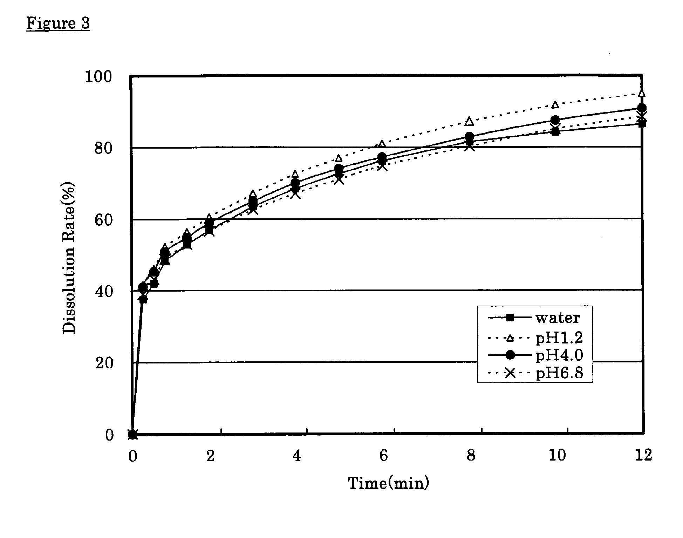

One tablet for the test was placed in 900 mL of each dissolution medium kept at the fluid temperature of 37 ± 0.5°C, a dissolution test was started at 50 rpm and 5 mL of dissolution medium was collected for every predetermined time and filtered through a membrane filter having a pore size of 0.45 µm to prepare a sample solution. The sample solution (5 µL) was subjected to a high-performance liquid chromatography (HPLC) to measure the dissolved amount of tramadol. The HPLC was conducted under the condition that a detector was an ultraviolet absorptiometer (measuring wavelength: 271 nm), a column was ODS (about 15 cm length x about 4 mm inner diameter), temperature of the column was about 40°C, a mobile phase was 0.05% trifluoroacetic acid/acetonitrile (75:25) and a flow rate was 1.0 mL/minute. The result until after 2 hours from the initiation of dissolution test using the tablets manufactured in the Example (100 mg tramadol hydrochloride/tablet) is shown in the graph of Fig. 1 and the result until after 2 hours from the initiation of dissolution test using the tablets manufactured in the Comparative Example is shown in the graph of Fig. 2. The result until after 12 hours from the initiation of dissolution test using the same tablets manufactured in the Example is shown in the graph of Fig. 3 and Table 2.

**Table 2**

| Dissolution Time (hr(s)) | Dissolution Rate (%) | | | |
|---|---|---|---|---|
| | Dissolution mediums | | | |
| | Water | pH 1.2 | pH 4.0 | pH 6.8 |
| 0 | 0.0 | 0.0 | 0.0 | 0.0 |
| 0.25 | 37.7 | 41.6 | 41.1 | 38.7 |
| 0.5 | 42.1 | 46.2 | 45.4 | 43.3 |
| 1 | 48.3 | 52.3 | 50.9 | 48.9 |
| 1.5 | 52.9 | 56.5 | 54.9 | 52.9 |
| 2 | 56.9 | 60.5 | 58.9 | 56.6 |
| 3 | 63.4 | 67.1 | 64.9 | 62.5 |
| 4 | 68.5 | 72.6 | 70.1 | 67.1 |
| 5 | 72.6 | 77.0 | 74.1 | 71.0 |
| 6 | 76.2 | 81.0 | 77.3 | 74.8 |
| 8 | 81.5 | 87.3 | 82.9 | 80.3 |
| 10 | 84.3 | 91.8 | 87.5 | 85.1 |
| 12 | 86.5 | 95.0 | 90.9 | 88.4 |

As apparent from the above-mentioned test results, when the solid pharmaceutical preparation of the present invention was subjected to a dissolution test according to the method 2 (Paddle method), Dissolution Test, General Tests, Processes and Apparatus of the Japanese Pharmacopoeia using 900 mL of a dissolution medium of 37°C fluid temperature at 50 rpm, tramadol hydrochloride of about 40% by weight, about 50% by weight, about 60% by weight, about 70% by weight and about 80% by weight was released after 15 minutes, 1 hour, 2 hours, 4 hours and 6 hours, respectively in each dissolution medium as shown in Table 2 whereupon its was shown that the solid pharmaceutical preparation of the present invention has a quick and preferred releasing characteristic. Accordingly, the solid pharmaceutical preparation of the present invention is a preparation where release of tramadol hydrochloride of 30 to 50% by weight, 40 to 60% by weight, 50 to 70% by weight, 60 to 80% by weight and 70% to 90% by weight after 15 minutes, 1 hour, 2 hours, 4 hours and 6 hours, respectively or, preferably, 35 to 45% by weight, 45 to 55% by weight, 55 to 65% by weight, 65 to 75% by weight or 75 to 85% by weight after 15 minutes, 1 hour, 2 hours, 4 hours and 6 hours, respectively is made possible in the above-mentioned dissolution test using each dissolution medium.

As a result of the use of a partly pregelatinized starch and a low substituted hydroxypropylcellulose as additives in a quick-release part of the solid pharmaceutical preparation of the present invention, an excellent initial dissolution behavior is achieved in various dissolution mediums having different pH values, tramadol which is an effective ingredient is quickly released and such a release is able to be maintained for long time the same as in the tablet products by a single-shot tabletting machine even when manufactured by a continuous tabletting machine for multiple layers. Moreover, the present preparation has a sufficient hardness in terms of strength and abrasion, breakage, crack, etc. of tablets were not generated in coating.

On the contrary, the product of the Comparative Example comprises a quick-release part where erythritol and crospovidone are used as main additives and, as shown in Fig. 2, the initial dissolution behavior varied depending upon pH of the solution when it was manufactured by a continuous tabletting machine. In addition, as compared with the case where the product was manufactured by a single-shot tabletting machine, the initial dissolution was significantly delayed. The preparation of the Comparative Example showed a quick and same initial dissolution behavior, even if pH of the dissolution medium is different when tabletting is coducted by a single-shot tabletting machine. However, when a scale-up was done and tabletting was conducted by a continuous tabletting machine, the above-mentioned disadvantage was apparent. As a result of various investigations, it was noted that such a disadvantage was generated by the fact that the sustained-release part is contaminated in the quick-release part due to adhesion and remaining of the sustained-release part to and in the inner area of the continuous tabletting machine.

### Industrial Applicability

As mentioned above, a two-layered solid pharmaceutical preparation containing tramadol hydrochloride which is one example of the solid pharmaceutical preparation of the present invention is a sustained-release preparation where an effective blood level is achieved immediately after administration for a quick mitigation of pain and the pharmaceutical effect may be sustained for long time. Since it has a quick releasing characteristic with little pH dependency in its initial dissolution, it has a very high usefulness as a sustained-release preparation which is able to achieve a stable drug level in blood without affection by variation and change of pH in digestive tract. Moreover, in the preparation of the present invention, stable and quick initial dissolution behavior being independent upon pH is achieved even when some sustained-release part is contaminated in quick-release part due to the difference in the tabletting method for multi-layered tablets. Furthermore, the preparation is practical as a preparation having a sufficient hardness in view of necessity that abrasion, breakage, crack, etc. are not generated when the tablet is coated.

## Claims

1. A solid pharmaceutical preparation having a quick-release part and a sustained-release part and containing a partly pregelatinized starch and a low substituted hydroxypropylcellulose as additives for the quick-release part.

2. The solid pharmaceutical preparation according to claim 1, wherein the partly pregelatinized starch is contained in an amount of 20 to 70% by weight to 100% by weight of the quick-release part.

3. The solid pharmaceutical preparation according to claim 1 or 2, wherein the low substituted hydroxypropylcellulose is contained in an amount of 5 to 25% by weight to 100% by weight of the quick-release part.

4. The solid pharmaceutical preparation according to any of claims 1 to 3, wherein synthetic aluminum silicate is further contained as an additive for the quick-release part.

5. The solid pharmaceutical preparation according to claim 4, wherein the synthetic aluminum silicate is contained in an amount of 1 to 15% by weight to 100% by weight of the quick-release part.

6. The solid pharmaceutical preparation according to any of claims 1 to 5, wherein an active analgesic ingredient is contained in the quick-release part and the sustained-release part.

7. The solid pharmaceutical preparation according to claim 6, wherein the active analgesic ingredient is tramadol or a pharmaceutically acceptable salt thereof.

8. The solid pharmaceutical preparation according to claim 6, wherein the active analgesic ingredient is tramadol hydrochloride.

9. The solid pharmaceutical preparation according to claim 8, wherein tramadol hydrochloride is contained in an amount of 15 to 70% by weight to 100% by weight of the quick-release part.

10. A solid pharmaceutical preparation having a quick-release part and a sustained-release part and containing tramadol hydrochloride, a partly pregelatinized starch and a low substituted hydroxypropylcellulose in the quick-release part in an amount of 20 to 55% by weight, 25 to 55% by weight and 5 to 20% by weight, respectively, to 100% by weight of the quick-release part.

11. The solid pharmaceutical preparation according to claim 10, wherein synthetic aluminum silicate is further contained as an additive for the quick-release part in an amount of 5 to 10% by weigh to 100% by weight of the quick-release part.

12. The solid pharmaceutical preparation according to any of claims 1 to 11, wherein it is a coated tablet.

13. In a solid pharmaceutical preparation containing tramadol or a pharmaceutically acceptable salt thereof in a quick-release part and a sustained-release part as an effective ingredient, a solid pharmaceutical preparation which is **characterized in that** a dissolution rate of the effective ingredient from said solid pharmaceutical preparation when a dissolution test is conducted by the method 2 (Paddle method) of Dissolution Test of General Tests, Processes and Apparatus of the Japanese Pharmacopoeia at fluid temperature of 37°C using 900 mL of a dissolution medium at 50 rpm is 30 to 50% by weight after 15 minutes, 40 to 60% by weight after 1 hour, 50 to 70% by weight after 2 hours, 60 to 80% by weight after 4 hours and 70 to 90% by weight after 6 hours.

14. The solid pharmaceutical preparation according to claim 13, wherein the dissolution rate of the effective ingredient is 35 to 45% by weight after 15 minutes, 45 to 55% by weight after 1 hour, 55 to 65% by weight after 2 hours, 65 to 75% by weight after 4 hours and 75 to 85% by weight after 6 hours.

15. The solid pharmaceutical preparation according to claim 13 or 14,
wherein the effective ingredient is tramadol hydrochloride.

16. The solid pharmaceutical preparation according to any of claims 13 to 15, wherein it is specified by a dissolution rate by a dissolution test using a dissolution medium of pH 1.2.
